(19) Europäisches Patentamt European Patent Office Office européen des brevets

(11) **EP 4 359 017 B1**

(12) **EUROPEAN PATENT SPECIFICATION**

(45) Date of publication and mention of the grant of the patent:
**23.04.2025 Bulletin 2025/17**

(21) Application number: **22741137.8**

(22) Date of filing: **22.06.2022**

(51) International Patent Classification (IPC):
**A61L 2/20** *(2006.01)* **A61L 2/22** *(2006.01)*
**A23B 7/144** *(2006.01)* **C01B 15/023** *(2006.01)*
**C01B 15/00** *(2006.01)*

(52) Cooperative Patent Classification (CPC):
**A61L 2/208; A23B 7/144; A61L 2/22; C01B 15/023;**
A61L 2202/23; A61L 2202/24; A61L 2202/25

(86) International application number:
**PCT/US2022/073075**

(87) International publication number:
**WO 2022/272257 (29.12.2022 Gazette 2022/52)**

(54) **A PROCESS FOR STERILIZATION TREATMENT USING A RESIDUE-FREE HYDROGEN PEROXIDE**

VERFAHREN ZUR STERILISATIONSBEHANDLUNG UNTER VERWENDUNG EINES RÜCKSTANDSFREIEN WASSERSTOFFPEROXIDS

PROCÉDÉ DE TRAITEMENT DE STÉRILISATION À L'AIDE D'UN PEROXYDE D'HYDROGÈNE SANS RÉSIDUS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **23.06.2021 EP 21181244**

(43) Date of publication of application:
**01.05.2024 Bulletin 2024/18**

(73) Proprietor: **Evonik Corporation Piscataway, NJ 08854 (US)**

(72) Inventors:
• **HUANG, Shurong Houston, Texas 77059 (US)**
• **AN, Weidong Williamsville, New York 14221 (US)**
• **KORZINEK, Pavel Manchester-by-the-sea, Massachusetts 01944 (US)**
• **ROVISON, JR., John M. New York 14132 (US)**

(74) Representative: **Evonik Patent Association c/o Evonik Industries AG IP Management Postcode 84/339 Rodenbacher Chaussee 4 63457 Hanau (DE)**

(56) References cited:
EP-A1- 1 762 252    EP-B1- 0 930 269
WO-A1-2015/008784   WO-A1-2018/142810
CN-A- 112 089 863   US-A- 5 232 680

**Description**

**Field of the invention**

The present invention relates to a process for sterilization treatment using a hydrogen peroxide vapour obtained from a non-stabilized aqueous hydrogen peroxide solution having a very low dry residue level.

**Background of the invention**

Hydrogen peroxide ($H_2O_2$) is the dominant chemical used for sterilization of material surfaces. Aseptic packaging of dairies and beverages and sterilization of medical devices are some examples of such applications. In the so-called Vapor Hydrogen Peroxide (VHP) aseptic decontamination process, the beverage containers, closures, and aseptic filling equipment are treated with a hydrogen peroxide vapour. The hydrogen peroxide solutions used for evaporation in this process must produce a very low dry residue in order to prevent incrustations in the evaporator or spraying equipment to avoid production downtime caused by frequent cleaning or damage of the equipment. The dry residues can originate primarily from the added stabilizers and to a lesser extent from the impurities present in the $H_2O_2$ solutions. On the other hand, hydrogen peroxide must be stable during production, transportation, storage and handling. In some cases, it should be possible to store $H_2O_2$ solution up to one year without any significant decomposition thereof. Such a high stability is usually achieved by addition of suitable stabilizers. These stabilizers are commonly non-volatile chemicals, which contribute to residue formation on vaporization. Much effort has been made to reduce the residue levels caused by the presence of stabilizers.

**Description of the prior art**

WO 2007031471 A1 discloses an aqueous hydrogen peroxide solution suitable for chemical sterilization of packaging materials comprising at least one stabilizer, wherein the said solution but without the stabilizer has a maximum phosphate content of 10 mg/kg and a dry residue at 105 °C of at most 10 mg/kg. Such a $H_2O_2$ solution feedstock was prepared by the anthraquinone process followed by a reverse osmosis purification using the membrane SWC3 at 28 bar. It is emphasized in this patent application that it is necessary to stabilize the thus obtained purified hydrogen peroxide solution with 10 mg / kg $H_2O_2$ solution of aminotrismethylene phosphonic acid prior to its use in spray aseptic packaging machines.

Similarly, WO 2015078830 A1 discloses aqueous solutions suitable for the chemical sterilization of packaging materials containing 10-50 wt% of hydrogen peroxide with a dry residue at 230 °C of at most 10 mg/kg (12-42 mg/kg at 105 °C) containing 10-50 ppm of citric acid as a stabilizer. The stabilized solution of $H_2O_2$ according to WO2015/078830A1 was shown to be stable for 12 months at 25 °C.

US20090175760A1 discloses 30-45 wt% hydrogen peroxide solutions suitable for vapour packaging sterilization, which contain no more than 2 ppb Fe, at least 15 ppb Al and no more than 40 ppm of a stabilizer comprising orthophosphoric acid. Stability tests performed at room temperature showed that a non-stabilized $H_2O_2$ solution containing no stabilizers and having an evaporation residue of < 1 ppm (comparative example 4) was much less stable than the inventive solutions containing orthophosphoric acid as a stabilizer and evaporation residues of 7-19 ppm.

WO 2018/142810 A1 describes a sterilization process using a hydrogen peroxide vapour made from an aqueous hydrogen peroxide solution having a dry residue of 10 ppm.

**Problem and solution**

It is well known from the prior art that minimizing dry residue of hydrogen peroxide solutions will improve the performance for vapour packaging sterilization. However, all relevant documents of the prior art explicitly teach to add various stabilizers in order to achieve appropriate stability of such $H_2O_2$ solutions, which inevitably increases the dry residue level of such stabilized solutions.

The inventors of the present patent application have surprisingly found that hydrogen peroxide solutions having dry residues at 105 °C of at most 5 ppm and typically containing no stabilizers, contrary to the teachings from the prior art, may be both stable and well suitable for using in vapour sterilization methods, if the content of some impurities, particularly transition metal ions, does not exceed a particular low level.

The object of the present invention is a process for sterilization or disinfection treatment, comprising the following steps:

a) obtaining a hydrogen peroxide vapour from an aqueous hydrogen peroxide solution, especially containing no added stabilizer, having a dry residue determined at 105 °C of at most 5 ppm and a total content of Fe, Cr, Mn and Ni in the solution of at most 6 ppb as determined by ICP-MS analysis;
b) treatment of an object to be sterilized or disinfected with a hydrogen peroxide vapour obtained in step a), wherein the treatment is preferably a sterilization or disinfection treatment.

**Hydrogen peroxide solution**

Aqueous hydrogen peroxide solution employed in the inventive process can be obtained by a well-known anthraquinone process (AO process), which generates hydrogen peroxide by hydrogenating a working solution of an alkylanthraquinone or an alkyltetrahydroanthraquinone in a water immiscible solvent and oxidizing the hydrogenated solution with molecular oxygen ($O_2$), usually with air. The hydrogen peroxide is then extracted with water from the oxidized working solution in an extraction column and the working solution is reused for generating hydrogen peroxide. Furthermore, the aqueous hydrogen peroxide solution can be concentrated in a distillation. An overview of the anthraquinone process is given in Ullmann's Encyclopedia of Industrial Chemistry, online edition, Vol. A18, pages 397-409, DOI 0.1002/14356007.a13_443.pub2, and in particular in Fig. 5 on page 401. A possible process for the purification of the $H_2O_2$ solution, e.g. that obtained by the above-described AO process, may include at least one further distillation, treatment with ion-exchange resin and/or reverse osmosis using a membrane. Examples of purification processes based on reversed osmosis can be found in the patent applications EP 0930269 A1 and WO 2005033005 A1. The exact purification procedure for obtaining the purified hydrogen peroxide solution to be employed in the inventive process should be selected based on the nature and concentrations of the impurities present in the used unpurified $H_2O_2$ solution. The main criterium of selecting the suitable production and purification method for a hydrogen peroxide solution is that it will provide an $H_2O_2$ solution having a dry residue at 105 °C of at most 5 ppm and a total content of Fe, Cr, Mn and Ni in the solution of at most 6 ppb, without containing any added stabilizer. The total content of Fe, Cr, Mn and Ni in the solution of at most 6 ppm belongs to the content of all mentioned metal species together. The metal analysis was performed by ICP-MS, which can detect any form of these metals in solution, therefore the metals do not have to be in elemental form, and metals in different forms will not affect the quantitative analysis results. However, preferably the metals are present as cations in hydrogen peroxide solution and can be detected accordingly.

The dry residue of the hydrogen peroxide solution determined at 105 °C, also referred to as "dry residue at 105 °C" in the present patent application, can be measured by the gravimetric method according to the following procedure, which is common in the field and is also similar to the procedure described in WO 2007031471 A1:

1. Place a platinum dish in an electric oven at 105 °C for 1h.
2. Cool the dish in a desiccator and then weigh to the nearest 0.0001 g; let this weight of the platinum dish be B [g].
3. Weigh to the nearest 0.01 g about 100g of a $H_2O_2$ sample in a 250 mL beaker and record the weight;
4. Transfer the $H_2O_2$ sample to a 250 mL addition funnel and reweigh the beaker to the nearest 0.01 g. The weight difference of the beaker before and after the sample addition will be W [g].
5. Drip the sample from the funnel into a platinum evaporating dish. Caution must be taken: the decomposition of hydrogen peroxide is accompanied by the production of substantial amount of heat and gas formation; sample must be added carefully.
6. Evaporate the solution carefully until dry, on an infrared lamp.
7. Place the dish in an oven at 105 °C for at least 1 hour.
8. Cool in a desiccator for 30 minutes and weigh the dish to the nearest 0.0001 g. Let this weight be A [g].
9. Calculate the dry residue at 105 °C using the equation :

$$\text{dry residue at } 105\ ^{\circ}\text{C [mg/kg = ppm]} = (A - B) \times 10^6 / W,$$

where:

- A (in g) is the weight of the dish containing the evaporation residue,
- B (in g) is the weight of the empty dish,
- W (in g) is the weight of the sample added to the dish.

The aqueous hydrogen peroxide solution employed in step a) of the process according to the invention has a dry residue determined at 105 °C, e.g. according to the method describe above, of at most 5 ppm, preferably at most 2 ppm. Most preferably, the dry residue at 105 °C is below the level detectable by this method, i.e. essentially no dry residue exceeding the error of the method can be determined by this method.

The non-volatile impurities in the employed hydrogen peroxide solution are usually in a lower ppb range. Some organic impurities may be present in the $H_2O_2$ solution in a lower ppm range, but they are usually either volatile or degradable during the evaporation, and therefore leave little residue after the hydrogen peroxide is vaporized.

Importantly, to achieve such a low dry residue levels, very low total amounts of $H_2O_2$ stabilizers, especially of non-volatile $H_2O_2$ stabilizers, e.g. at most about 5 ppm, preferably at most about 3 ppm, more preferably at most about 1 ppm, more preferably at most about 0.5 ppm, more preferably no such $H_2O_2$ stabilizers at all, should be present or be added to the solution.

The term "non-volatile" refers in the context of the present invention to the components, which cannot be vaporized at 105 °C, i.e. remain at least partly in the dry residue obtained after drying of the residue at 105 °C as described above.

The hydrogen peroxide solution used in the inventive process preferably contains no $H_2O_2$ stabilizers at all, neither the volatile, nor the non-volatile ones. Unnecessary stabilizer chemicals may be harmful, increasing the residue level and accelerating corrosion.

The term "$H_2O_2$ stabilizer" refers to any additive added to slow down the $H_2O_2$ decomposition. Such $H_2O_2$ stabilizers of various types are well known from the prior art.

Metal ions, particularly transition metal ions can initiate hydrogen peroxide decomposition. Activity of the different metal ions varies significantly; some metal impurities may act synergistically. Since the most equipment for producing of hydrogen peroxide solution is made of stainless steel, the primary metal impurities to initiate peroxide decomposition are believed to be the components of the stainless steel such as iron (Fe), chromium (Cr), nickel (Ni), manganese (Mn) and copper (Cu).

The total content of Fe, Cr, Mn and Ni in the hydrogen peroxide solution employed in the inventive process is at most 6 ppb, preferably at most 4 ppb, more preferably at most 2 ppb, as determined by Inductively Coupled Plasma - Mass Spectrometry (ICP-MS) analysis. The ICP-MS method is known to those skilled in the art to provide reliable analysis results for determining of very low metal concentrations in the solution.

The Cu content in the employed hydrogen peroxide solution is preferably at most 0.2 ppb, more preferably at most 0.1 ppb, more preferably at most 0.05 ppb, as determined by ICP-MS analysis.

The Cr content in the employed hydrogen peroxide solution is preferably at most 3 ppb, more preferably at most 2 ppb, more preferably at most 1.5 ppb, as determined by ICP-MS analysis.

The Fe content in the employed hydrogen peroxide solution is preferably at most 2 ppb, more preferably at most 1 ppb, more preferably at most 0.5 ppb, as determined by ICP-MS analysis.

The Mn content in the employed hydrogen peroxide solution is preferably at most 1 ppb, more preferably at most 0.5 ppb, more preferably at most 0.1 ppb, as determined by ICP-MS analysis.

The Ni content in the employed hydrogen peroxide solution is preferably at most 2 ppb, more preferably at most 1 ppb, more preferably at most 0.5 ppb, as determined by ICP-MS analysis.

The total content of all metals in the employed hydrogen peroxide solution is preferably at most 20 ppb, more preferably at most 15 ppb, more preferably at most 10 ppb, as determined by ICP-MS analysis.

The ppm and ppb values are calculated defined as weight data.

The usual construction materials for the equipment suitable for storage and use of the hydrogen peroxide solution before and during the inventive process include specific polymers and metals. For long-term storage of $H_2O_2$ solutions used in the inventive process, polymers such as high-density polyethylene (HDPE) and polytetrafluoroethylene (PTFE) are most suitable. Aluminum, preferably of types 1060, 1100, 5254 and stainless steel, preferably of types 304, 304L, 316 and 316L can be used for pieces of equipment that will have a short contact time with the peroxide, such as pumps, valves, or small buffer containers. Glass can be used as a suitable construction material in certain cases, e.g. for lab experiments or stability tests.

The hydrogen peroxide solution used in the inventive process preferably has an active oxygen loss of no more than 4 wt-%, preferably of no more than 2 wt-%, as determined after heating the solution for 24 hours at 100 °C in a glass vessel.

The hydrogen peroxide solution employed in the process of the invention is preferably suitable for storing for at least four days, more preferably for at least 5 days, more preferably for at least 7 days in an appropriate stainless steel (e.g. of types 304, 304L, 316 and/or 316L) or aluminum (e.g. aluminium of types 1060, 1100, and/or 5254) container at 20- 30 °C temperature, preferably under room temperature of 20-22 °C, most preferably under room temperature of 20 °C with a peroxide concentration drop of less than 0.1 wt%.

Hydrogen peroxide concentration drop can be determined according to the ISO 7157 by a titration method.

The hydrogen peroxide solution used in the inventive process preferably contains 10% to 50%, more preferably 20% to 45%, more preferably 30% to 40%, by weight of hydrogen peroxide. The hydrogen peroxide content can be measured according to the ISO 7157 by a titration method.

The acidity of the solution (as $H_2SO_4$ equivalent) in the invention usually does not exceed 3 ppm, preferably not more than 1 ppm.

The used hydrogen peroxide solution can have a pH of 2.0-4.5, preferably 3.0-4.0.

The aqueous $H_2O_2$ solution employed in the inventive process preferably presents a conductivity of from 0.5 to 90

μS/cm, more preferably from 2 to 50 μS/cm, more preferably from 5 to 10 μS/cm. The conductivity can be measured according to the method of the standard DIN IEC 60746-1. The conductivity of the employed hydrogen peroxide solution is low and can be adjusted by adding nitric acid when higher conductivity is needed. The conductivity of the hydrogen peroxide solutions can be roughly increased by 3 μS/cm per ppm nitric acid addition.

Preferably, the performed sterilization and/or disinfection treatment of an object is performed for a few seconds, more preferably 1-20 seconds, most preferred 2-6 seconds.

**Sterilization and disinfection method**

The inventive process can advantageously be used for different types of sterilization and disinfection treatments, particularly for the chemical sterilization of packaging materials by hydrogen peroxide vapor in aseptic packaging processes.

The object to be sterilized can preferably be selected from the group consisting of packaging materials such as beverage and dairy containers, cartons, bottles, caps and other closures, as well as vessels, aseptic and other cold-fill shelf-stable filling equipment and machinery, ambulance or rooms, and medical devices. The inventive method is not limited to sterilization. The objects such as fruits and vegetables can also be disinfected with the vaporized hydrogen peroxide by the inventive process.

In the inventive process, the hydrogen peroxide vapor can be produced by evaporation or vaporization of the hydrogen peroxide solution. Evaporation or vaporization of the $H_2O_2$ solution can be achieved e.g. under normal (1 atm), reduced (< 1 atm) or increased (> 1 atm) pressure optionally followed by spraying thereof onto the object to be sterilized. $H_2O_2$ solution can be heated, e.g. to at least 50 °C prior to or during the evaporation. Evaporation or vaporization of the hydrogen peroxide solution can also be affected by passing a stream of a carrier gas, e.g. air or nitrogen through the evaporator or vaporizer.

The particular advantage of the employed $H_2O_2$ solution for the inventive process resides in the combination of (1) the high stability of the hydrogen peroxide solution and (2) very low dry residue of this $H_2O_2$ solution.

As a result, the process of the invention can be carried out for at least 100 hours, preferably for at least 500 hours, more preferably for at least 1000 hours without the need to clean the residue after obtaining the hydrogen peroxide vapour in step a) of the process. In most cases, no cleaning at all will be required for a usual operation time of the continuously used evaporation equipment before the usual maintenance service, e.g. once in a year thereof. The residue referees to any non-volatile impurities left in the heaters. It can coat on the heating surfaces, decrease the heat transfer rate, corrode the surface and contaminate customer's products. So the residue must be removed periodically. Chemical and mechanical cleanings are usually performed. The cleaning can cause a major production down time, and damage the expensive heating parts.

**Brief description of the drawings**

Figure 1 shows dry residue obtained after evaporation of 100 g of an unstabilized residue-free hydrogen peroxide solution according to the invention (sample #1).

Figure 2 shows dry residue obtained after evaporation of 100 g of a commercially available stabilized premium hydrogen peroxide solution for $H_2O_2$ vapor applications.

**Examples**

**Example 1**

Samples #1-#7 of 35% wt% unstabilized hydrogen peroxide solutions with low metal contents were prepared by the known from the prior art purification methods of hydrogen peroxide solution prepared from the anthraquinone process, such as by ion-exchange resin treatment, distillation and/or reversed osmosis.

The decomposition rate of 35% wt% unstabilized hydrogen peroxide solutions in HDPE bottles with a vented cap at room temperature (20 °C) was measured by determining the assay drop and the $H_2O_2$ loss. The assay drop was the differential decrease of hydrogen peroxide concentration after 30 days. the $H_2O_2$ loss was the decrease of hydrogen peroxide in 100% basis from 100g of the test solution after 30 days. The dependence of the hydrogen peroxide decomposition rate on impurity concentrations can be seen from the comparison of the test results in Tables 1a and 1b.

**Table 1a: Impurity effect on decomposition rate of $H_2O_2$ solutions with the low impurity level.**

| Samples | Assay drop | $H_2O_2$ Loss, g | Metals Impurities in $H_2O_2$ Solutions, ppb | | | | | |
|---|---|---|---|---|---|---|---|---|
| | | | Cu | Cr | Fe | Mn | Ni | Total |
| #1 | -0.02% | -0.02 | 0.00 | 1.29 | 0.12 | 0.01 | 0.06 | 1.48 |
| #2 | -0.03% | -0.03 | 0.00 | 1.24 | 0.09 | 0.00 | 0.03 | 1.36 |
| #3 | -0.03% | -0.04 | 0.00 | 1.11 | 0.07 | 0.00 | 0.01 | 1.19 |
| #4 | -0.02% | -0.03 | 0.00 | 0.59 | 0.15 | 0.01 | 0.05 | 0.80 |
| #5 | -0.02% | -0.03 | 0.01 | 1.05 | 0.34 | 0.03 | 0.22 | 1.64 |
| #6 | -0.01% | -0.04 | 0.00 | 0.75 | 0.13 | 0.01 | 0.06 | 0.95 |
| #7 | -0.01% | -0.04 | 0.00 | 0.63 | 0.06 | 0.00 | 0.01 | 0.70 |

**Comparative Example 1**

Samples #8-#14 of 35% wt% unstabilized hydrogen peroxide solutions with higher metal contents were prepared by the known from the prior art purification methods of hydrogen peroxide solution prepared from the anthraquinone process by ion-exchange resin treatment, distillation and/or reversed osmosis.

The decomposition rate of the 35% wt% unstabilized hydrogen peroxide solutions in HDPE bottles with a vented cap at room temperature (20 °C) was measured by determining the assay drop and the $H_2O_2$ loss after 30 days of storage. The results are summarized in Table 1b.

**Table 1b: Impurity effect on decomposition rate of $H_2O_2$ solutions with the higher impurity level.**

| $H_2O_2$ Sample | Assay drop | $H_2O_2$ Loss, g | Metals Impurities in $H_2O_2$ Solutions, ppb | | | | | |
|---|---|---|---|---|---|---|---|---|
| | | | Cu | Cr | Fe | Mn | Ni | Total |
| #8 | -0.18% | -0.22 | 0.09 | 3.62 | 3.91 | 0.30 | 2.50 | 10.33 |
| #9 | -0.22% | -0.27 | 0.07 | 1.87 | 3.31 | 0.18 | 1.54 | 6.90 |
| #10 | -0.16% | -0.19 | 0.08 | 2.45 | 3.13 | 0.25 | 1.94 | 7.77 |
| #11 | -0.16% | -0.20 | 0.13 | 3.30 | 3.22 | 0.29 | 2.39 | 9.20 |
| #12 | -0.23% | -0.28 | 0.07 | 2.17 | 4.06 | 0.20 | 1.62 | 8.05 |
| #13 | -0.32% | -0.39 | 0.09 | 3.10 | 4.85 | 0.31 | 2.50 | 10.76 |
| #14 | -0.35% | -0.40 | 0.07 | 2.07 | 4.21 | 0.21 | 1.59 | 8.08 |

It can be seen from the results that increase in the metal impurity level of the unstabilized $H_2O_2$ solutions from the samples in Table 1a to those of Table 1b led to a significant increase in the decomposition rate of the latter samples.

**Example 2**

The starting hydrogen peroxide solution for sample #6 was stored in HDPE bottles with a vented cap under three different temperatures: 20, 35 and 50 °C, respectively. The decomposition rates of these unstabilized hydrogen peroxide solutions (samples #15-20) at the specified temperatures were measured in HDPE bottles with a vented cap, for each tested sample twice, by determining the assay drop and the $H_2O_2$ loss after 1st 30 and 2nd 30 days of storage, respectively.

The results are shown in Table 2. It was observed that there was no problem to store this unstabilized hydrogen peroxide solution under slightly elevated temperature. The hydrogen peroxide concentration after 30 days remained almost unchanged after the storage at 20 °C (positive values of the assay drop), and actually increased under elevated temperatures, due to the evaporation of the solution. $H_2O_2$ loss even after 30 days of storage at 50 °C was still below 1% (1g per 400 g) of the solution. The decomposition rates of the peroxide in the second 30 days test was similar to the first 30 days test.

**Table 2: Temperature effect on decomposition rates of a $H_2O_2$ solution (precursor for sample #6) with a low impurity level**

| $H_2O_2$ Sample | #15 | #16 | #17 | #18 | #19 | #20 |
|---|---|---|---|---|---|---|
| Temp. °C | 20 | | 35 | | 50 | |
| Changes in 30 days | | | | | | |
| Wt Loss, g | -0.01 | -0.01 | -0.80 | -1.03 | -3.39 | -3.47 |
| Assay Drop | -0.01% | -0.01% | 0.01% | 0.03% | 0.07% | 0.11% |
| $H_2O_2$ loss, g | -0.04 | -0.06 | -0.25 | -0.23 | -0.88 | -0.77 |
| Changes in another 30 days | | | | | | |
| Wt Loss, g | -0.02 | -0.02 | -0.81 | -1.10 | -3.44 | -3.54 |
| Assay Drop | 0.00% | -0.01% | 0.01% | 0.04% | 0.07% | 0.10% |
| $H_2O_2$ loss, g | -0.01 | -0.04 | -0.24 | -0.24 | -0.91 | -0.84 |

**Example 3**

The starting hydrogen peroxide solution for sample #6 was exposed to SS (stainless steel) coupons in glass Kjeldahl flasks. Both glassware and coupons were passivated by following industry's standards. 110 g solution was in contact with each coupon (2" x 0.5" x 0.125") at 20 °C initially and the samples were analysed twice: after a 4-days period and then after a 7-days period (total of 11 days). Then the samples (#22a and 22b) were moved into an incubator at 35 °C, and kept in it for another 4-days period before a third analysis for the samples.

Additionally, a stabilized hydrogen peroxide solution sample #21 was prepared by adding 1ppm of amino tris(methylenephosphonic acid) (ATMP), a common $H_2O_2$ stabilizer, to the same starting solution as for sample #6 and tested under the same conditions as the unstabilized samples #22a/#22b. Blank samples (the same peroxide solutions to samples #21a/#21b and #22a/#22b but without SS coupons) were also run in parallel as a reference.

The decomposition rates of the unstabilized samples #22a/#22b and the stabilized samples #21a/#21b at the specified temperatures and times are summarized in Table 3.

**Table 3: Stainless steel and stabilizer effect on the decomposition rates of $H_2O_2$ solutions.**

| Sample | Blank to #21 | #21a | #21b | Blank to #22 | #22a | #22b |
|---|---|---|---|---|---|---|
| description | with 1ppm ATMP | | | Unstabilized | | |
| Decompositions in initial 4 days at 20°C | | | | | | |
| wt loss, g | 0.00 | 0.00 | 0.00 | 0.00 | -0.04 | -0.03 |
| Assay drop | 0.01% | 0.01% | 0.01% | 0.01% | -0.04% | -0.06% |
| $H_2O_2$ loss, g | 0.01 | 0.01 | 0.01 | 0.01 | -0.06 | -0.07 |
| Decompositions in next 7 days after initial 4 days at 20 °C | | | | | | |
| wt loss, g | 0.00 | -0.02 | -0.03 | -0.03 | -0.09 | -0.12 |
| Assay drop | 0.03% | -0.04% | -0.02% | 0.03% | -0.24% | -0.22% |
| $H_2O_2$ loss, g | 0.02 | -0.06 | -0.04 | 0.01 | -0.21 | -0.19 |
| Decompositions in the last 4 days at 35 °C | | | | | | |
| wt loss, g | 0.01 | -0.63 | -0.57 | -0.02 | -0.42 | -0.43 |
| Assay drop | 0.01% | -1.19% | -1.04% | 0.00% | -0.83% | -0.66% |
| $H_2O_2$ loss, g | 0.02 | -1.22 | -1.07 | -0.01 | -0.85 | -0.70 |

Stainless steel is the primary construction material for aseptic filling machines. The accumulative contact time of the hydrogen solution with stainless steel in these machines can be up to four days under ambient conditions and even longer.

When both solutions were in contact with stainless steel coupons, the peroxide solution with 1 ppm ATMP was slightly more stable for the first 11 days under room temperature (20-22 °C). However, after the samples were moved into an incubator and stored for another 4 days at 35°C, the hydrogen peroxide containing 1 ppm ATMP surprisingly started to decompose even faster than the sample without ATMP. The ATMP in the peroxide might promote more metal leaching than stabilizing the metals.

**Example 4**

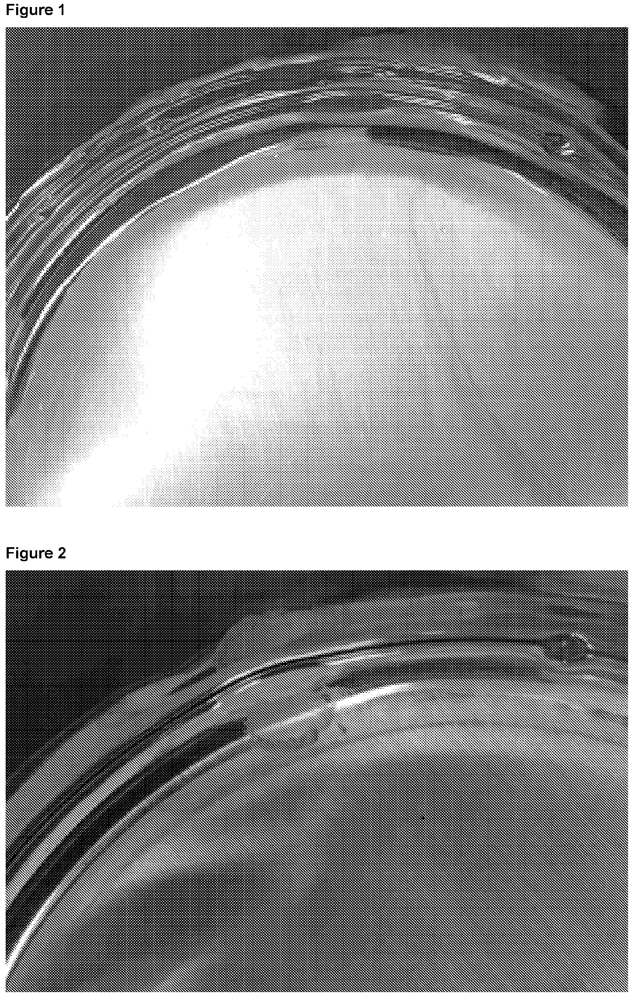

Unstabilized 35% wt% hydrogen peroxide solution (100 g) from the starting solution of sample #6 was slowly evaporated in a Pt crucible according to steps 1-9 of the method described in the description above. Almost no residue could be visually observed in the crucible, as shown in Figure 1. The residue value was too low to be quantitatively determined (below 2 ppm).

**Comparative example 4**

The residue test described in example 4 was repeated using 100 g of a commercially available stabilized premium hydrogen peroxide solution for vapor applications. A significant amount of a residue was formed from the hydrogen peroxide as can be seen in Figure 2.
The presented examples show that the hydrogen peroxide solutions containing no stabilizers, and having a relatively low level of metal impurities, are sufficiently stable during the storage under ambient conditions. They can be handled with regular equipment (SS pumps, meters, pipes) and analysed in a regular lab (no cleanroom is needed). They will produce little residues on evaporation and so they are well suitable for using in evaporation processes, such as sterilization or disinfection treatments with a H$_2$O$_2$ vapour, without the need to often remove the dry residue formed after the evaporation.

**Claims**

1.   A process for a sterilization or disinfection treatment, comprising the following steps:

     a) obtaining a hydrogen peroxide vapour from an aqueous hydrogen peroxide solution containing no stabilizer and having a dry residue determined at 105 °C of at most 5 ppm and a total content of Fe, Cr, Mn and Ni in the solution of at most 6 ppb as determined by ICP-MS analysis;
     b) sterilization or disinfection treatment of an object to be sterilized or disinfected with a hydrogen peroxide vapour obtained in step a).

2.   The process according to claim 1, wherein the dry residue at 105 °C from the hydrogen peroxide solution is below the level detectable by this method.

3.   The process according to claim 1 or 2, wherein the total content of all non-volatile hydrogen peroxide stabilizers present in the aqueous hydrogen peroxide solution is at most 1 ppm, preferably is essentially zero.

4.   The process according to any of claims 1 to 3, wherein the total content of Fe, Cr, Mn and Ni in the hydrogen peroxide solution is at most 4 ppb as determined by ICP-MS analysis.

5.   The process according to any of claims 1 to 4, wherein the Cu content in the hydrogen peroxide solution is at most 0.2 ppb, preferably at most 0.1 ppb, as determined by ICP-MS analysis.

6.   The process according to any of claims 1 to 5, wherein the content of all metals in the hydrogen peroxide solution is at most 20 ppb, as determined by ICP-MS analysis.

7.   The process according to any of claims 1 to 6, wherein the hydrogen peroxide solution has an active oxygen loss of no more than 4 wt-%, preferably of no more than 2 wt-%, as determined after heating the solution for 24 hours at 100 °C in a glass vessel.

8.   The process according to any of claims 1 to 7, wherein the hydrogen peroxide solution is suitable for storing for at least four days in an appropriate stainless steel or aluminium container at 20-30 °C temperature with a peroxide concentration drop of less than 0.1 wt%.

9. The process according to any of claims 1 to 8, wherein the hydrogen peroxide solution has a pH of 2.0-4.5, preferably 3.0-4.0.

10. The process according to any of claims 1 to 9, wherein the hydrogen peroxide solution is obtained by the anthraquinone process followed by purification thereof by at least one distillation, treatment with ion-exchange resin and/or reverse osmosis using a membrane.

11. The process according to any of claims 1 to 10, wherein the hydrogen peroxide solution contains 10% to 50%, preferably 20% to 45%, more preferably 30% to 40%, by weight of hydrogen peroxide.

12. The process according to any of claims 1 to 11, wherein the hydrogen peroxide vapor is produced by evaporation or vaporization of the hydrogen peroxide solution.

13. The process according to any of claims 1 to 12, wherein the object to be sterilized or disinfected is selected from the group consisting of packaging materials such as beverage and dairy containers, cartons, bottles, caps and other closures, as well as vessels, aseptic and other cold-fill shelf-stable filling equipment and machinery, objects such as fruit and vegetables, medical devices, contained space like ambulance or rooms.

14. The process according to any of claims 1 to 13, wherein step b) of the process is vapour aseptic packaging process carried out at a temperature of at least 50 °C of the object to be sterilized or disinfected.

**Patentansprüche**

1. Verfahren für eine Sterilisations- oder Desinfektionsbehandlung umfassend die folgenden Schritte:

a) Erhalten eines Wasserstoffperoxiddampfs aus einer wässrigen Wasserstoffperoxidlösung, die keinen Stabilisator enthält und einen Trockenrückstand, bestimmt bei 105 °C, von höchstens 5 ppm und einen Gesamtgehalt an Fe, Cr, Mn und Ni in der Lösung von höchstens 6 ppb, wie bestimmt durch ICP-MS-Analyse, aufweist;
b) Sterilisations- oder Desinfektionsbehandlung eines zu sterilisierenden oder desinfizierenden Gegenstandes mit einem bei Schritt a) erhaltenen Wasserstoffperoxiddampf.

2. Verfahren nach Anspruch 1, wobei der Trockenrückstand bei 105 °C aus der Wasserstoffperoxidlösung unter dem Nachweisniveau dieses Verfahren liegt.

3. Verfahren nach Anspruch 1 oder 2, wobei der Gesamtgehalt an allen in der wässrigen Wasserstoffperoxidlösung vorhandenen nichtflüchtigen Wasserstoffperoxidstabilisatoren höchstens 1 ppm beträgt, vorzugsweise im Wesentlichen Null beträgt.

4. Verfahren nach einem der Ansprüche 1 bis 3, wobei der Gesamtgehalt an Fe, Cr, Mn und Ni in der Wasserstoffperoxidlösung höchstens 4 ppb, wie bestimmt durch ICP-MS-Analyse, beträgt.

5. Verfahren nach einem der Ansprüche 1 bis 4, wobei der Cu-Gehalt in der Wasserstoffperoxidlösung höchstens 0,2 ppb beträgt, vorzugsweise höchstens 0,1 ppb, wie bestimmt durch ICP-MS-Analyse.

6. Verfahren nach einem der Ansprüche 1 bis 5, wobei der Gehalt aller Metalle in der Wasserstoffperoxidlösung höchstens 20 ppb, wie bestimmt durch ICP-MS-Analyse, beträgt.

7. Verfahren nach einem der Ansprüche 1 bis 6, wobei die Wasserstoffperoxidlösung einen Verlust an aktivem Sauerstoff von nicht mehr als 4 Gew.-% aufweist, vorzugsweise von nicht mehr als 2 Gew.-%, wie bestimmt nach Erhitzen der Lösung für 24 Stunden auf 100 °C in einem Glasgefäß.

8. Verfahren nach einem der Ansprüche 1 bis 7, wobei die Wasserstoffperoxidlösung zur Lagerung für wenigstens vier Tage in einem geeigneten Edelstahl- oder Aluminiumbehälter bei 20-30 °C Temperatur mit einem Abfall der Peroxidkonzentration von weniger als 0,1 Gew.-% geeignet ist.

9. Verfahren nach einem der Ansprüche 1 bis 8, wobei die Wasserstoffperoxidlösung einen pH-Wert von 2,0-4,5, vorzugsweise 3,0-4,0, aufweist.

10. Verfahren nach einem der Ansprüche 1 bis 9, wobei die Wasserstoffperoxidlösung erhalten ist durch das Anthrachinonverfahren, gefolgt von Reinigung davon durch wenigstens eine Destillation, Behandlung mit Ionenaustauscherharz und/oder Umkehrosmose unter Verwendung einer Membran.

11. Verfahren nach einem der Ansprüche 1 bis 10, wobei die Wasserstoffperoxidlösung 10 Gew.-% bis 50 Gew.-%, vorzugsweise 20 Gew.-% bis 45 Gew.-%, bevorzugter 30 Gew.-% bis 40 Gew.-%, Wasserstoffperoxid enthält.

12. Verfahren nach einem der Ansprüche 1 bis 11, wobei der Wasserstoffperoxiddampf durch Abdampfen oder Verdampfen der Wasserstoffperoxidlösung erzeugt wird.

13. Verfahren nach einem der Ansprüche 1 bis 12, wobei der zu sterilisierende oder desinfizierende Gegenstand ausgewählt ist aus der Gruppe bestehend aus Verpackungsmaterialien, wie z.B. Getränke- und Milchbehälter, Kartons, Flaschen, Kappen und anderen Verschlüssen, sowie Gefäßen, aseptischen und anderen Geräten und Maschinen zur lagerstabilen Kaltabfüllung, Gegenständen wie Obst und Gemüse, medizinischen Vorrichtungen, umgegrenztem Raum wie Krankenwagen oder Räume.

14. Verfahren nach einem der Ansprüche 1 bis 13, wobei Schritt b) des Verfahrens bei einer Temperatur von wenigstens 50 °C durchgeführtes dampfaseptisches Verpackungsverfahren des zu sterilisierenden oder desinfizierenden Gegenstands ist.

## Revendications

1. Procédé pour un traitement de stérilisation ou de désinfection, comprenant les étapes suivantes :

   a) obtention d'une vapeur de peroxyde d'hydrogène à partir d'une solution aqueuse de peroxyde d'hydrogène ne contenant pas de stabilisant et ayant un résidu à sec déterminé à 105 °C d'au plus 5 ppm et une teneur totale en Fe, Cr, Mn et Ni dans la solution d'au plus 6 ppb, comme déterminée par analyse ICP-MS ;
   b) traitement de stérilisation ou désinfection d'un objet à stériliser ou désinfecter avec une vapeur de peroxyde d'hydrogène obtenue à l'étape a).

2. Procédé selon la revendication 1, dans lequel le résidu à sec à 105 °C de la solution de peroxyde d'hydrogène est inférieur au niveau détectable par ce procédé.

3. Procédé selon la revendication 1 ou 2, dans lequel la teneur totale en tous les stabilisants de peroxyde d'hydrogène non volatils présents dans la solution aqueuse de peroxyde d'hydrogène est d'au plus 1 ppm, de préférence est essentiellement nulle.

4. Procédé selon l'une quelconque des revendications 1 à 3, dans lequel la teneur totale en Fe, Cr, Mn et Ni dans la solution de peroxyde d'hydrogène est d'au plus 4 ppb, comme déterminée par analyse ICP-MS.

5. Procédé selon l'une quelconque des revendications 1 à 4, dans lequel la teneur en Cu dans la solution de peroxyde d'hydrogène est d'au plus 0,2 ppb, de préférence d'au plus 0,1 ppb, comme déterminée par analyse ICP-MS.

6. Procédé selon l'une quelconque des revendications 1 à 5, dans lequel la teneur de tous les métaux dans la solution de peroxyde d'hydrogène est d'au plus 20 ppb, comme déterminée par analyse ICP-MS.

7. Procédé selon l'une quelconque des revendications 1 à 6, dans lequel la solution de peroxyde d'hydrogène a une perte d'oxygène actif de pas plus de 4 % en poids, de préférence de pas plus de 2 % en poids, comme déterminée après chauffage de la solution pendant 24 heures à 100 °C dans un récipient en verre.

8. Procédé selon l'une quelconque des revendications 1 à 7, dans lequel la solution de peroxyde d'hydrogène est appropriée pour être stockée pendant au moins quatre jours dans un récipient approprié en acier inoxydable ou en aluminium à une température de 20 à 30 °C avec une chute de concentration en peroxyde inférieure à 0,1 % en poids.

9. Procédé selon l'une quelconque des revendications 1 à 8, dans lequel la solution de peroxyde d'hydrogène a un pH de 2,0 à 4,5, de préférence de 3,0 à 4,0.

**10.** Procédé selon l'une quelconque des revendications 1 à 9, dans lequel la solution de peroxyde d'hydrogène est obtenue par le procédé à l'anthraquinone suivi d'une purification de celle-ci par au moins une distillation, un traitement avec une résine échangeuse d'ions et/ou une osmose inverse en utilisant une membrane.

**11.** Procédé selon l'une quelconque des revendications 1 à 10, dans lequel la solution de peroxyde d'hydrogène contient 10 % à 50 %, de préférence 20 % à 45 %, plus préférablement 30 % à 40 %, en poids de peroxyde d'hydrogène.

**12.** Procédé selon l'une quelconque des revendications 1 à 11, dans lequel la vapeur de peroxyde d'hydrogène est produite par évaporation ou vaporisation de la solution de peroxyde d'hydrogène.

**13.** Procédé selon l'une quelconque des revendications 1 à 12, dans lequel l'objet à stériliser ou désinfecter est choisi dans le groupe constitué des matériaux d'emballage tels que des récipients pour boissons et produits laitiers, des cartons, des bouteilles, des bouchons et autres fermetures, ainsi que des récipients, des équipements et machines de remplissage longue conservation aseptiques et autres à remplissage à froid, des objets tels que des fruits et légumes, des dispositifs médicaux, des espaces confinés tels que des ambulances ou des chambres.

**14.** Procédé selon l'une quelconque des revendications 1 à 13, dans lequel l'étape b) du procédé est un procédé d'emballage aseptique à vapeur effectué à une température d'au moins 50 °C de l'objet à stériliser ou à désinfecter.

**Figure 1**

**Figure 2**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2007031471 A1
- WO 2015078830 A1
- US 20090175760 A1
- WO 2018142810 A1
- EP 0930269 A1
- WO 2005033005 A1

**Non-patent literature cited in the description**

- Ullmann's Encyclopedia of Industrial Chemistry, vol. A18, 397-409